# EUROPEAN PATENT APPLICATION

(11) **EP 4 215 919 A1**
(43) Date of publication of application: **26.07.2023**
(21) Application number: 22152242.8
(22) Date of filing: 19.01.2022
(51) Int. Cl.: G01N 33/94

(54) **METHOD OF DETECTING ENDOGENOUS CANNABINOIDS IN BLOOD AND USES THEREOF**

(71) Applicant: UNIVERSITÄTSmedizin der Johannes Gutenberg- Universität Mainz, 55131 Mainz (DE)
(72) Inventor: Bindila, Laura Maria, 55130 Mainz (DE); Lerner, Raissa, 55116 Mainz (DE); Schwitter, Claudia, 67596 Frettenheim (DE)
(74) Representative: Grahn, Sibylla Maria

(57) **Abstract**

The present invention relates to method of detecting endocannabinoids from dried blood spots. The present invention further relates to a method for diagnosing and/or monitoring a disease or condition which is related to a change of the concentration of endocannabinoid(s) in the blood or plasma, which comprises the detection method. The present invention further relates to a method for control testing.

## Description

The present invention relates to method of detecting endocannabinoids from dried blood spots. The present invention further relates to a method for diagnosing and/or monitoring a disease or condition which is related to a change of the concentration of endocannabinoid(s) in the blood or plasma, which comprises the detection method. The present invention further relates to a method for control testing.

### BACKGROUND OF THE INVENTION

In clinical and preclinical research, the investigation of the endogenous cannabinoid levels in relation to health, disease development, and therapies, as well as of the effects of cannabis treatment and/or recreational use on endogenous cannabinoid levels is typically carried out by measuring the circulating endogenous cannabinoid levels in plasma or serum of human subjects.

Thereby, blood has to be sampled by venipuncture by qualified medical personnel and requires further processing to plasma or serum by centrifugation. In order to avoid ex-vivo, artificial changes and sample-to-sample variability of the endogenous cannabinoid levels, controlled temperature and time for centrifugation, aliquotation, and time between blood sampling and plasma/serum isolation, and/or the use of chemical and enzyme stabilizers is required for reliable comparative clinical and preclinical studies. Moreover, the isolated plasma/serum has to be snap frozen and then stored (at -20°C and/or -80°C) within a short and pre-defined time period, and then transported from the collection institution to the analytical laboratory on dry ice (approx. -20°C).

Accordingly, this procedure incurs significant costs for trained personnel, consumables and instrumentation for blood processing, plasma/serum isolation, storage, and transportation, as well as significant processing time. Typically, circulating endogenous (and exogenous) cannabinoids are extracted and analyzed in specified, often distinct laboratories and/or by distinct methodological conditions. Endocannabinoids and exogeneous cannabis are typically analyzed from different sample aliquots. Such practice renders, apart from above mentioned financial and logistical burden, also concerns for inter- and intra-study reproducibility, and variability in correlating exogenous and endogenous cannabinoid levels in a physiological or pathophysiological context from distinctly prepared and analyzed blood specimens.

It is an objective of the present invention to provide improved means and methods for analyzing endogenous cannabinoids from patient blood samples.

### SUMMARY OF THE INVENTION

According to the present invention this object is solved by a method of detecting endocannabinoids in blood, comprising the steps of
(a) providing a dried blood spot (DBS) obtained from the blood of a subject; preferably in a DBS collection card,
(b) cutting the dried blood spot or piece(s) thereof and collect it into a sample tube;
(c) adding extraction solvents and an internal standard to the sample tube,
   wherein at first a solution of 300 µl ethylacetate/n-hexane in a ratio of 9:1 (v/v) containing further the internal standard are added followed by 300 µl 0.1 M formic acid,
(d) vortexing the mixture in the sample tube overnight while cooling; preferably at about 4°C for about 20 hours,
(e) separating the organic phase from the aqueous phase by centrifuging at 20,000xg for about 10 min while cooling,
   preferably at about 4°C,
(f) placing the sample tube on dry ice or in a freezer and letting the aqueous phase freeze; preferably for about 10 to 20 min, such as 15 min,
(g) transferring the organic phase to a new sample tube while cooling; preferably at about 4°C,
(h) evaporating organic solvents under a N₂ stream and obtaining a dried lipid extract;
(i) reconstituting the dried lipid extract in acetonitrile/water at a ratio of 1:1 (v/v), vortexing the mixture and transferring it to a sample tube suitable for mass spectrometry; and
(j) analyzing the sample mixture obtained in step (i) by performing targeted mass spectrometry using liquid chromatography multiple reaction monitoring (LC/MRM) analysis and simultaneous identifying endocannabinoid(s) and/or metabolite(s) thereof and/or endocannabinoid-related lipid(s).

According to the present invention this object is solved by a method for diagnosing and/or monitoring a disease or condition or disorder, which is related to a change of the concentration of endocannabinoid(s) and/or metabolite(s) thereof and/or endocannabinoid-related lipid(s) in the blood or plasma,
comprising the method of the present invention, wherein the DBS is obtained from the blood of a patient or subject having said disease or condition or disorder or is suspected of having said disease or condition or disorder.

According to the present invention this object is solved by a method for control testing for cannabis use,
comprising the detection method of the present invention, wherein the DBS is obtained from the blood of a subject which is tested for diagnosis, monitoring and/or therapy response by forensic psychology, medical bodies, clinical researchers, psychologists, and/or psychiatrists.

### DESCRIPTION OF THE PREFERRED EMBODIMENTS OF THE INVENTION

Before the present invention is described in more detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art. For the purpose of the present invention, all references cited herein are incorporated by reference in their entireties.

Concentrations, amounts, and other numerical data may be expressed or presented herein in a range format. It is to be understood that such a range format is used merely for convenience and brevity and thus should be interpreted flexibly to include not only the numerical values explicitly recited as the limits of the range, but also to include all the individual numerical values or sub-ranges encompassed within that range as if each numerical value and sub-range is explicitly recited. As an illustration, a numerical range of " 1 to 20" should be interpreted to include not only the explicitly recited values of 1 to 20, but also include individual values and sub-ranges within the indicated range. Thus, included in this numerical range are individual values such as 1, 2, 3, 4, 5 .... 17, 18, 19, 20 and sub-ranges such as from 2 to 10, 8 to 15, etc. This same principle applies to ranges reciting only one numerical value, such as "higher than 150 mg per day". Furthermore, such an interpretation should apply regardless of the breadth of the range or the characteristics being described.

### Detection method for endocannabinoids

As outlined above, the present invention provides a method of detecting endocannabinoids in blood.

Said detection method comprises the steps of
(a) providing a dried blood spot (DBS) obtained from the blood of a subject;
(b) cutting the dried blood spot or piece(s) thereof and collect it into a sample tube;
(c) adding extraction solvents and an internal standard to the sample tube,
   wherein at first a solution of 300 µl ethylacetate/n-hexane in a ratio of 9:1 (v/v) containing further the internal standard are added followed by 300 µl 0.1 M formic acid,
(d) vortexing the mixture in the sample tube overnight while cooling;
(e) separating the organic phase from the aqueous phase by centrifuging at 20,000xg for about 10 min while cooling,
(f) placing the sample tube on dry ice or in a freezer and letting the aqueous phase freeze;
(g) transferring the organic phase to a new sample tube while cooling;
(h) evaporating organic solvents under a N₂ stream and obtaining a dried lipid extract;
(i) reconstituting the dried lipid extract in acetonitrile/water at a ratio of 1:1 (v/v), vortexing the mixture and transferring it to a sample tube suitable for mass spectrometry; and
(j) analyzing the sample mixture obtained in step (i) by performing targeted mass spectrometry using liquid chromatography multiple reaction monitoring (LC/MRM) analysis and simultaneous identifying endocannabinoid(s) and/or metabolite(s) thereof and/or endocannabinoid-related lipid(s).

### Step (a)

In step (a), a dried blood spot (DBS) obtained from the blood of a subject is provided.

Preferably, the dried blood spot is in a DBS collection card.

DBS collection cards are known in the art and are commercially available. In a preferred embodiment, the DBS collection card is not pre-treated.

For example, a Whatman^{®} FTA^{®} DMPK-C card is used (Merck KGaA, Darmstadt, Germany).

DBS microvolume sampling requires only 10 to 20 µl per sample. Preferably, a full blood spot on the FTA^{®} DMPK-C is to be collected.

The detection method allows remote or self-blood sampling.

In one embodiment, the dried blood spot is obtained at the point-of-care, at home.

In one embodiment, the dried blood spot is obtained in hospitals, clinical research laboratories, blood collection points, medical practices, psychiatry, psychology practices.

The dried blood spot can be obtained at any blood collection points for research and development or clinical diagnostic and monitoring - provided the drying, storage and transport conditions are accordingly followed.

### Step (b)

In step (b), the dried blood spot or piece(s) thereof are cut and collected into a sample tube.

Preferably,
- the entire dried blood spot is cut out, preferably when the spot is entirely full (which is equal to 20 µl blood),
- a piece of 2 mm × 3 mm of the dried blood spot is cut out, or
- a piece of 1 mm × 6 mm of the dried blood spot is cut out.

Preferably, with a razor sharp cutting edge or similar devices where controlled diameters are cut-out from the DBS spot.

In one embodiment, the cut out dried blood spot or piece(s) thereof are further cut into pieces and then collected in the sample tube.

### Step (c)

In step (c), extraction solvents and internal standards are added to the sample tube.

The extraction solvents are a solution of 300 µl ethylacetate/n-hexane in a ratio of 9:1 (v/v), which are added first, followed by 300 µl 0.1 M formic acid.

The solution of 300 µl ethylacetate/n-hexance in a ratio of 9:1 (v/v) contains the internal standard.

Preferably, the internal standard used in step (c) comprises a mixture of endo- and exocannabinoids, which are preferably deuterated.

Said mixture preferably comprises
2-arachidonoyl glycerol (2-AG),
anandamide (AEA),
1-arachidonoyl glycerol (1-AG),
arachidonic acid (AA),
oleoylethanolamine (OEA),
palitoylethanolamine (PEA),
cannabidiol (CBD),
delta-9-tetrahydrocannabinol (THC),
11-hydroxy-delta-9-tetrahydrocannabinol (OH-THC), and
1 1-nor-carboxy-delta-9-tetrahydro-cannabinol (THCA).

More preferably, said mixture comprises
2-arachidonoyl glycerol (2-AG)-d5,
anandamide (AEA)-d4,
1-arachidonoyl glycerol (1-AG)-d5,
arachidonic acid (AA)-d8,
oleoylethanolamine (OEA)-d4,
palitoylethanolamine (PEA)-d4,
cannabidiol (CBD)-d3,
delta-9-tetrahydrocannabinol (THC)-d3,
II-hydroxy-delta-9-tetrahydrocannabinol (OH-THC)-d3, and
1 1-nor-carboxy-delta-9-tetrahydro-cannabinol (THCA)-d3.

### Step (d)

In step (d), the mixture in the sample tube is vortexed overnight while cooling.

Preferably, step (d) is carried out at about 4°C, such as in a cold room or thermomixer, for about 20 hours

### Step (e)

In step (e), the organic phase is separated from the aqueous phase by centrifuging at 20,000xg for about 10 min while cooling.

Preferably, step (e) is carried out at about 4°C, such as in a cold room.

The organic phase contains the lipids, the aqueous phase contains the proteins.

### Step (f)

In step (f), the sample tube is placed on dry ice or at -20°C in a freezer. Thereby the aqueous phase freezes.

Preferably, step (f) is carried out for about 10 to 20 min, such as 15 min.

### Step (g)

In step (g), the organic phase is transferred to a new sample tube while cooling.

Preferably, step (g) is carried out at about 4°C, such as in a cold room or by using pre-cooled sample tubes, or on a cooled surface.

In a preferred embodiment step (d), (e) and/or (g) are carried out at 4°C such as in a coldroom or a cooled surface.

### Step (h)

In step (h), the organic solvents are evaporated under a N₂ stream and a dried lipid extract is obtained.

Preferably, step (h) is carried out at about 37°C.

In one embodiment, the dried lipid extract obtained in step (h) is stored at about -20°C before carrying out steps (i) and (j).

### Step (i)

In step (i), the dried lipid extract is reconstituted in acetonitrile/water at a ratio of 1:1 (v/v), the mixture is vortexed and transferred to a sample tube suitable for mass spectrometry.

A sample tube suitable for mass spectrometry is for example a glass tube or glass vial.

For large sample cohorts, (siliconized) microtiter or deep well plates are preferably used.

### Step (j)

In step (j), the sample mixture obtained in step (i) is analyzed by performing targeted mass spectrometry using liquid chromatography multiple reaction monitoring (LC/MRM) analysis.

In step (j), endocannabinoid(s) and/or metabolite(s) thereof and/or endocannabinoid-related lipid(s) can be detected in a simultaneous manner.

Preferably, the endocannabinoids are 2-arachidonoyl glycerol (2-AG), anandamide (AEA), 1-arachidonoyl glycerol (1-AG).

Preferably, the metabolites of endocannabinoids are arachidonic acid (AA).

Preferably, the endocannabinoid-related lipids are oleoylethanolamine (OEA), palitoylethanolamine (PEA).

Preferably, performing targeted mass spectrometry using liquid chromatography multiple reaction monitoring (LC/MRM) analysis in step (j) comprises:
- preparing calibration curves,
- preparing quality control (QC) sample,
- setting LC/MRM conditions,
   including LC gradient, flow rate, injection volume and conditions, ion source and detection parameters, MRM transitions in the instrument's method page,
- equilibrating instrument with the said LC/MRM method and running QC sample,
- writing sample batch,
- placing samples and calibrants into the autosampler,
- running batch analysis,
- removing left-over samples and evaporating, or applying lid and storing for additional or replicate analysis,
- quantifying data using quantification software provided with the instrument,
- normalizing data to the blood volume, in an external quantification sheet (excel or any other tool of choice),
- performing statistical analysis.

Said targeted mass spectrometry using LC/MRM is described in further detail below.

In a preferred embodiment, the detection method of the present invention further comprises step (k):
(k) determining the amount of the endocannabinoid(s) and/or metabolite(s) thereof and/or endocannabinoid-related lipid(s).

In a preferred embodiment, the detection method of the present invention further comprises step (1):
(1) identifying exocannabinoid(s), and optionally determining their amount.

Preferably, the exocannabinoids are cannabidiol (CBD), delta-9-tetrahydrocannabinol (THC), 1 1-hydroxy-delta-9-tetrahydrocannabinol (OH-THC), 11-nor-carboxy-delta-9-tetrahydro-cannabinol (THCA).

In a preferred embodiment, the subject is
a patient having a disease or condition which is related to a change of the concentration of endocannabinoid(s) and/or metabolite(s) thereof and/or endocannabinoid-related lipid(s) in the blood or plasma or is a subject suspected of having said disease or condition; or
a subject exhibiting behavioral or psychological disorders and/or behavioral changes which are related to a change of the concentration of endocannabinoid(s) and/or metabolite(s) thereof and/or endocannabinoid-related lipid(s) in the blood or plasma or is a subject suspected of having said disorders.

Said disease or condition or disorder which is related to a change of the concentration of endocannabinoid(s) and/or metabolite(s) thereof and/or endocannabinoid-related lipid(s) in the blood or plasma is preferably a neurological disorder, a psychological or psychiatric disorder, behavioral disorder, eating and/or metabolic disorders.

Preferably, the neurological disorder is selected from epilepsy and neurodevelopmental diseases, such as Tourette syndrome.

Preferably, the psychological or psychiatric or behavioral disorder is selected from anxiety, stress-related disorders, post-traumatic stress disorder, addiction, and attention deficit hyperactivity disorder (ADHD).

### Medical uses

As outlined above, the present invention provides a method for diagnosing and/or monitoring a disease or condition or disorder which is related to a change of the concentration of endocannabinoid(s) and/or metabolite(s) thereof and/or endocannabinoid-related lipid(s) in the blood or plasma.

Said method comprises the detection method of the present invention.

In said method, the DBS is obtained from the blood of a patient or subject having said disease or condition or disorder or is suspected of having said disease or condition or disorder.

Said disease or condition or disorder is preferably as defined herein above.

In one embodiment, the monitoring of the disease or condition or disorder comprises monitoring the treatment of said disease or condition or disorder and/or monitoring the course or progress of said disease or condition or disorder,
wherein preferably the detection method of the present invention is repeated over time.

### Control uses

As outlined above, the present invention provides a method for control testing for cannabis use.

Said method for control testing for cannabis use is carried out in patients with a disease or condition or disorder related to a change in endocannabinoids and/or related endocannabinoid lipids and treatment thereof.

Said method comprises the detection method of the present invention.

In said method, the DBS is obtained from the blood of a subject which is tested for diagnosis, monitoring and/or therapy response by forensic psychology, medical bodies, clinical researchers, psychologists, or psychiatrists.

### Further description of preferred embodiments

As discussed above, in clinical and preclinical research, the investigation of the endogenous cannabinoid, named endocannabinoids, levels in relation to health, disease development, and therapies, as well as of the effects of cannabis treatment and/or recreational use on endogenous cannabinoid levels is typically carried out by measuring the circulating endogenous cannabinoid levels in plasma or serum of human subjects. Thereby, blood has to be sampled by venipuncture by qualified medical personnel and requires further processing to plasma or serum by centrifugation. In order to avoid ex-vivo, artificial changes and sample-to-sample variability of the endogenous cannabinoid levels, controlled temperature and time for centrifugation, aliquotation, and time between blood sampling and plasma/serum isolation, and/or the use of chemical and enzyme stabilizers is required for reliable comparative clinical and preclinical studies. Moreover, the isolated plasma/serum has to be snap frozen and then stored (at -20°C and/or -80°C) within a short and pre-defined time period, and then transported from the collection institution to the analytical laboratory on dry ice (approx. -20°C). Accordingly, this procedure incurs significant costs for trained personnel, consumables and instrumentation for blood processing, plasma/serum isolation, storage, and transportation, as well as significant processing time. Typically, circulating endogenous cannabinoids are extracted and analyzed in specified, often distinct laboratories than the ones analyzing cannabis (exogenous cannabinoids) and/or by distinct methodological conditions, and from different sample aliquots. Such practice renders, apart from above mentioned financial and logistical burden, also concerns for inter- and intra-study reproducibility, and variability in correlating exogenous and endogenous cannabinoid levels in a physiological or pathophysiological context from distinctly prepared and analyzed blood specimens. Moreover, in studies were endogeneous levels are of interest in patients and/or subjects, the use of exogeneous cannabis is typically assessed by selfreporting. Therefore, co-analysis of endo- and exocannabinoids from the same sample, particularly DBS sample, can increase the confidence in the evaluation of endocannabinoid regulation with disease, condition or disorder, by omitting the bias due to possible false selfreporting of cannabis use.

These limitations are fully addressed by the method(s) of the present disclosure which was developed for blood endogenous cannabinoid extraction and analysis, with the possibility to co-extract and analyze exogeneous cannabinoids, based on dry blood spot (DBS) blood sampling, extraction and targeted mass spectrometric analysis, e.g. liquid-chromatography / multiple reaction monitoring (LC/MRM) as follows:
*Sampling:* blood sampled on the DBS cards (approx. 20 µL per DBS spot) is a suitable and viable source for endogenous cannabinoid extraction and analysis by targeted mass spectrometry (targeted mass spectrometry using LC/MRM is the preferred method of choice for exogenous and endogenous cannabinoids analyses).

Due to the low blood volume needed for this assay, a higher flexibility of blood sampling for such analyses is attained including:
i) finger-prick DBS sampling, as the most common DBS sampling method,
ii) when blood sampled by venipuncture is of interest for medical research or tests, a following blood spotting on the DBS cards is readily attainable and allows endogenous cannabinoid analyses without compromising the blood volume intended for other medical investigation and/or without the need for extra blood sampling,
iii) DBS spotting from a blood biobank,
iv) blood spotting at the anatomical site of a surgical intervention, and/or peripheral blood sampling in surgery room without the extra steps for blood-to-plasma/serum processing,
v) samples remotely collected, for example at home-collection, or on- forensic site sampling (if here endocannabinoids are of interest to be investigated),
vi) samples collected at a psychology and/or psychiatry practice or hospital,
vii) any other form of generating blood drops is suitable.

This method allows also blood sampling in live animals by cheek or tail puncture for time-course analysis of endogenous cannabinoids, and/or provision of blood droplets following animal sacrifice, again, without the extra-steps of blood-to-plasma/serum processing.

Therefore, the here described DBS sampling facilitates blood analysis of endocannabinoids and prevents the extra steps of plasma/serum processing. Also exocannabinoids can be co-extracted and analysed at the same time.

*Processing:* Due to the fact that DBS collected blood is suitable to extract and analyze endocannabinoids, the need for instrumentation and consumables for plasma and serum isolation, such as on-site or near-site centrifugation, specific vials compatible with plasma/serum isolation and storage, as well as tools and means to maintain a constant temperature condition, i.e. 4°C during these procedures are prevented. Additionally, the time constrain for blood-to-plasma processing, e.g. minutes, which imposes substantial personnel and logistic efforts, particularly in hospitals, is alleviated when using the method presented here. Essentially, all these steps are replaced in our method by a simple drying of the DBS cards for 3-4 hours at room temperature followed by storage.

*Storage:* Given that endocannabinoid levels are stable at 4 °C on DBS cards up to one week, and at -20°C or -80°C for one week to months, a major logistic and financial challenge in hospitals is addressed, that is, it prevents the need to have immediate post-sampling access to freezer (-20°C or -80°C), such as the case when plasma/serum is processed (see above). Thus, it allows the sampling facilities to store the DBS cards at 4°C (up to one week) and/or a more flexible time frame to transport the samples to the long-term storage facilities (-20°C or -80°C), biobanks, analytical laboratory, etc. The space for storage of DBS cards is significantly reduced compared to storing plasma/serum samples in vials. This is valid both for the sampling facilities and the analytical laboratory which extracts and quantifies the endocannabinoids. Additionally, due to the fact that the present method allows for remote blood sampling in human subjects, a subsequent home storage at 4°C (less than a week) or -20°C (more than a week) prior to shipping to the laboratory or hospital is afforded.

*Sample preparation and extraction of endocannabinoids:* Endocannabinoid levels are typically normalized to the plasma/serum volume. For this purpose, either an accurate aliquotation of the isolated plasma/serum, or an accurate up-take of a fixed volume of serum/plasma from the storage tubes is first necessary in a classical approach to endocannabinoid analysis from plasma/serum. This step is substantially less time-consuming and tedious using DBS cards, since the capacity per DBS spot is pre-defined and equal from spot to spot.

A further advantage of this protocol is that it permits co-extraction of exogenous and endogenous cannabinoids, and all the more, from low blood volumes, e.g. sampled on DBS cards. Thus, not only the sampling and blood processing is facilitated, but the blood expenditure reduced, while the time-, cost- and sample handling effectiveness for extraction is increased.

*Analysis:* The multiplex analytical method (e.g. LC/MRM) for endogenous (and exogenous) cannabinoid analysis, which is a single experiment, is in itself advantageous for facilitating lower sample consumption for their qualitative and quantitative determination with the added benefits of targeted mass spectrometry (e.g. selectivity, accuracy, robustness, high-throughput), and it is further applicable for various biological matrices tissues, cells, biological fluids, etc.

The suitability of this method for endocannabinoids, metabolites thereof, the endocannabinoid-related lipids and exocannabinoids investigation from DBS cards provides an integrated solution, e.g. *"one blood drop, one analysis",* of high overall sensitivity, and multiplex data provision of endo- and exocannabinoids and their interplay for clinical and preclinical research and management.

Profiling blood levels of endocannabinoids has become of great interest in clinical and preclinical research for a broad spectrum of diseases, including cardiorespiratory, immune and metabolic diseases, mental, psychiatric and behavioral disorders, respectively, and healthy development, etc. on one hand. On another hand, the use of phytocannabinoids in clinical management of diseases, but also as adjuvants and/or therapeutic replacements in self-therapy has been increasingly pursued. In this context, the modulation of the endogenous cannabinoid system by the different exogenous cannabinoid preparations and its consequential correlation with disease course or healthy development is not yet entirely understood, warranting further investigations and profiling for prospective clinical decision and health policy makers. Moreover, changes induced in the circulating levels of endocannabinoids upon recreational use of phytocannabinoids and their correlates with health development and prospective on-set of diseases is a pivotal monitoring requisite for discovery of life quality and health consequences, prophylactic disease management, and accordingly, adjusting guidelines for clinical and recreational use of phytocannabinoids.

The method described here allows the profiling and determination of circulating levels of endogenous and exogenous cannabinoids from the same blood sample collected on dried blood spots, in a single analytical step, and through a single extraction step. Combined with the multiplex analysis of both, endogenous and exogenous cannabinoids facilitates the discovery of the consequences of phytocannabinoids use/administration on the levels of the endogenous cannabinoids, as well as their correlates with healthy development, disease onset and progression, or therapy response (see above). Moreover, for endogenous cannabinoid-centric (pre)clinical studies, the co-analysis of exogenous cannabinoids in blood can be used as a quality control of phytocannabinoids use, preventing thus possible erroneous self-reports of recreational phytocannabinoids use, and hence increasing specificity of the endogenous cannabinoid-based health and disease profiling and monitoring.

Noteworthy, a blood-biological assay for both endogenous and exogenous cannabinoids obtainable from dried blood spots is expected to expedite clinical studies by:
i) facilitating large-scale and longitudinal studies through increased accessibility to blood samples, including remote or self-sampling, without the need of stationary or ambulant administration in the hospital at every time point for the study,
ii) allowing cost-effective and standardized remote or professional blood sampling, biobanking, transport, processing and analysis,
iii) allowing multiplexed blood assay (single extraction and analysis) for both endogenous and exogenous cannabinoids, hence reducing variability occurring otherwise in distinct analyses,
iv) increased intra- and inter-laboratory reproducibility of analysis by preventing variability due to sampling, type of consumables for plasma/serum isolation, storage and transport, and
v) v) increased reliability of comparative studies across and between laboratories due to multiplexing the assay and increased standardization of sampling, biobanking, processing and analysis.

The method of the present invention can be used in the following ways:
*Preclinical research laboratories:* Research laboratories and hospitals who want to carry out longitudinal studies of cannabis use and its effect on endogenous cannabinoids can highly benefit by the biological assay presented herein, as it allows live sampling of blood from animal models, e.g. blood withdrawal from live animals is sufficient for DBS storage and subsequent extraction/quantification of exogenous and endogenous cannabinoids. The same advantages are applicable for human studies, whereby hospitals, preclinical research laboratories can more flexibly and reliably design and implement large-scale, time-course longitudinal studies in humans, at lower costs, higher standardization and throughput and increased accessibility to blood samples (including self and remote sampling).

*Clinical research, clinical trials:* Clinical research and concurrently clinical trials on the therapeutic effects of different preparation of phytocannabinoids are increasingly conducted to improve therapeutic management and clinical decision. In this context, analyses of endocannabinoids or co-analyses of endo- and exocannabinoids from low blood volumes collected on dried blood spots will benefit not only the throughput, cost-effectiveness and readily implementation and standardization but also cross-study comparison and reference of the interplay between circulating phytocannabinoid and endocannabinoid levels in relation to a disease and/or therapy.

*Pharmaceutical industry:* Different formulations and extracts of phytocannabinoids, as well as different modes of administrations are being developed for therapeutic purposes by pharmaceutical companies. For such applications both the circulating levels, metabolic products, and the half-time of the exogenous cannabinoids, as well as their effect on the circulating levels of endogenous cannabinoids require testing. For such purposes, the assay disclosed herein will facilitate such studies and tests, again, also due to the benefits of increased throughput, standardization, cost-effectiveness, reliability of comparative studies, and inter-and intra-laboratory reproducibility potential.

*Unreported cannabis use:* Psychologists, psychiatrists, medical professionals, clinical researchers that have to rely on self-report cannabis use due to the current lack of on-site sampling/measuring tools of cannabis use could implement such a DBS procedure, which can then be sent to an analytical laboratory for determination. Moreover, blood sampling for researching the endocannabinoid system in psychiatric and psychological disorders, also in relation to cannabis use, can be implemented in such clinical psychology and/or psychiatry facilities to expedite the development of precise diagnosis and therapeutic strategies.

### Protocol 1: Preparation of dried blood spots (DBS) and extraction of endocannabinoids

### Material for finger prick blood collection:

- Sterile lancet
- DBS collection card (i.e. FTA DMPK-C cards)
- Alcohol 70-90% for sterilisation
- Gloves

### Procedure:

- Sterilize the side of blood collection on the finger with alcohol
- Let alcohol dry
- Puncture the side of the finger with a sterile lancet
- Touch the blood droplet formed to the surface and in the center of each spot of the card
- Let the spot dry for 3-4 h until spots have a homogeneous brown color (workbench)
- For long term storage it is recommended to wrap the cards in tissue paper and place them in clean zipper bags (1-5 cards per bag) and store at - 20° or 80 °C

### Caution: Do not touch the surface of the DBS spots prior to or after the blood collection

### Extraction of endocannabinoids from DBS for LC-MRM-MS/MS analysis:

- clean scissors with i.e. isopropanol before usage
- cut out the whole DBS with scissors following the broken line of the spot, if the spot is full, or use razor sharp cutting edge with 3 or 6 mm diameter, where possible. 3 mm razor sharp cutting edge is to be used for spots which are less than full, for example for about 10 µL blood.
- cut each spot in about 4 pieces directly into an 1.5 or 2 ml Eppendorf tube. Make sure the cut pieces are small enough to fit in the 1.5 or 2 mL Eppendorf tube
- add 300 µL of ethylacetate/n-hexane (9:1; v/v, containing a mixture of endo- and exocannabinoid internal standards (see also Protocol 2 below)
- add 300 µL of 0.1 M formic acid
- vortex mix in cold room (~4 °C) over night
- centrifuge 10 min (4°C, 20000 g)
- place the tubes on dry ice or at -20°C in a freezer and let freeze the aqueous phase (~15 min)
- transfer upper organic phase in new 1.5 ml Eppendorf tube
- evaporate organic phase under a gentle stream of N₂, typically at 37°C
- reconstitute residue in 50 µL ACN/water (1:1; v/v), vortex mix for ~ 2 min and transfer into glass inserts for LC-MRM analysis. If the LC/MRM analysis cannot be done immediately after extraction, please store the dried extracts (e.g. after evaporation) at -20°C until analysis. Avoid repeated thawing cycles.
- run the LC/MRM analysis according to the SOP for LC MRM analysis 2017

Internal standard types and preparation for endocannabinoids are described in Bindila and Lutz (2016).

Internal standards for exocannabinoids preparation for DBs can be found in published literature.

### Protocol 2: LC-MRM: Analysis of endocannabinoids, exocannabinoids, arachidonic acid, palmitoylethanolamine and oleoylethanolamine

The LC/MRM method for endocannabinoid quantification is published in Bindila and Lutz (2016). Validation data of the LC/MRM method for quantification of endocannabinoids, arachidonic acid, palmitoylethanolamine and oleoylethanolamine is published in Lerner *et al.* (2017).

### 1) Materials and methods

Note: All solutions for analyte extraction and LC/MRM analysis have to be of LC/MS grade purity. Use only freshly prepared solutions.

### a) Internal and calibration standards for all analytes

1. Calibration standards: Anandamide (AEA), 2-arachidonoyl glycerol (2-AG), arachidonic acid (AA), oleoylethanolamine (OEA), palmitoylethanolamine (PEA), 2-arachidonoyl glycerol (1-AG), cannabidiol (CBD), delta-9-tetrahydrocannabinol (THC), 11-hydroxy-delta-9-tetrahydrocannabinol (OH-THC), 11-nor-9-carboxy-delta-9-tetrahydrocannabinol (THCA).

Aliquot the original samples in acetonitrile and store at -20°C. 2. Deuterated standards: AEA-d4, 2-AG-d5, AA-d8, OEA-d4, PEA-d4, 1-AG-d5, CBD-d3, THC-d3, OH-THC-d3, THCA-d3.

### b) LC/MRM instrumentation and accessories

1. LC solvents: Solvent A 0.1% formic acid in water; solvent B 0.1 % formic acid in acetonitrile.
2. LC glass vials with siliconized inserts (*see* Note 1 below).
3. LC column: 2.5 µm C18(2)-HST column, 100 mm × 2 mm, combined with a precolumn (C18, 4 mm × 2 mm).
4. Autosampler maintained at 4°C.
5. LC instrument.
6. 5500 QTrap triple-quadrupole linear ion trap mass spectrometer equipped with a Turbo V Ion Source (AB SCIEX) and polarity switching mode or another triple quadrupole mass spectrometer.
7. LC quality control sample: Mix deuterated eCB standards and standard eCBs to an equimolar mixture and in acetonitrile/water (1:1, v/v). Store at -20°C before use. LC quality control sample can also be a calibration point mixture (calibration solution 4 or 5).
8. Acetonitrile/water solution (1:1, v/v), prepare fresh and maintain at 4°C.
9. Acetonitrile/isopropanol solution (1:1, v/v), prepare fresh and maintain at 4°C.

### 2. LC/MRM analysis procedure

1. Calibration curve solutions. Prepare fresh, on the day of analysis a mixture of the calibration standards in acetonitrile in a LC glass vial. Pipette increasing volumes of this calibration standard mixture into LC glass vials in order to create a calibration curve. Add 50 µL of spiking solution of internal standards to each vial and complement with appropriate volume of acetonitrile up to 250 µL or 500 µL and then add 250 µl or 500 µL of water in each vial to make up for 500 µl or 1 mL each calibration solution, respectively.
2. Accurately pipette cold 50 µL acetonitrile/water (1:1, v/v) into the tubes containing the dry lipid extracts.
3. Vortex for 30 sec at room temperature.
4. Centrifuge for 3 min at 10,000 g at 4°C. Centrifuge has to be pre-cooled at 4 °C.
5. Transfer 30 µL of the sample solutions from tubes into LC glass inserts, placed into LC glass vials and close with open lid caps.
6. Place the sample-containing LC vials and the vials containing calibration standards into autosampler maintained at 4°C.
7. Set following conditions for LC/MRM method (*see* Note 2):
   LC gradient: increase solvent B (acetonitrile containing 0.1% formic acid) over 5 min from 55% to 90%, and maintain it at 90% for 4 min, decrease over 0.5 min to 55% and
   maintain for 0.5 min at 55%.
   LC flow rate: 300 µL/min.
   LC injection volume: 20 µL; use pre- and post-syringe cleaning.
   Ion source: +4800 V for positive ion mode, -4500 V for negative ion mode; curtain gas:
      40; temperature (TEM): 550 °C; ion source gas 1 and 2: 50; Polarity switching time: 50ms

MRM transitions in positive ion mode: AEA, *m*/*z* 348.3 to *m*/*z* 62.1; AEA-d4, *m*/*z* 352.3 to *m*/*z* 62.1; 2-AG, *m*/*z* 379.1 to *m*/*z* 287.2; 2-AG-d5, *m*/*z* 384.2 to *m*/*z* 287.2; PEA, *m*/*z* 300.2 to *m*/*z* 62.1; PEA-d4, *m*/*z* 304.2 to *m*/*z* 62.1; OEA, *m*/*z* 326.2 to *m*/*z* 62.1; OEA-d4, *m*/*z* 330.2 to *m*/*z* 62.1; CBD, *m*/*z* 315.2 to *m*/*z* 193.0; CBD-d3, *m*/*z* 318.2 to *m*/*z* 196.0; THC, *m*/*z* 315.2 to *m*/*z* 193.0; THC-d3, *m*/*z* 318.2 to *m*/*z* 196.0; THCA, *m*/*z* 345.0 to *m*/*z* 299.0; THCA-d3, *m*/*z* 348.0 to *m*/*z* 302.0; OH-THC, *m*/*z* 331.0 to *m*/*z* 193.0; OH-THC-d3, *m*/*z* 334.0 to *m*/*z* 196.

Compound parameters in positive ion mode: EP: 10 V; dwell time: 20 ms; DP: for AEA, AEA-d4, 2-AG, 2-AG-d5, OEA, OEA-d4, PEA, PEA-d4 70 V, for CBD 61 V, for CBD-d3 116 V, for THC, THC-d3, THCA, THCA-d3, OH-THC and OH-THC-d3 80 V; CE: for AEA, AEA-d4, 2-AG, 2-AG-d5 22 V, for OEA, PEA, PEA-d4 21 V, for OEA-d4 23 V, for CBD, CBD-d3, THC, THC-d3, THCA, THCA-d3, OH-THC and OH-THC-d3 30 V; CXP: for AEA, AEA-d4, THC, THC-d3, THCA, THCA-d3, OH-THC and OH-THC-d3 9 V, for 2-AG, 2-AG-d5 30 V, for OEA, OEA-d4, PEA, PEA-d4 10 V, for CBD and CBD-d3 12 V.

MRM transitions negative ion mode: AA, *m*/*z* 303.05 to *m*/*z* 259.1; AA-d8, *m*/*z* 311.04 to *m*/*z* 267.0.

Compound parameters in negative ion mode: EP: -10 V; dwell time: 50 ms; DP: -200 V; CE: - 19 V; CXP: -12 V.

8. Equlibrate the LC/MRM system with the instrument default method for equilibration, for 2-5 min.

9. Equilibrate LC/MRM system with the method for eCBs analysis for 5-10 min

10. Verify the performance of the LC/MRM using the LC quality control sample

11. Write a sample batch for LC/MRM analysis. A minimum of three calibration curves are necessary: one at the beginning of the batch, one in the middle of the bacth and one at the end of the batch. Place a wash run before and after every calibration curve. For the wash run inject 20 µL of acetonitrile/isopropanol (1:1, v/v). Run regularly LC quality control samples (it can also be a calibration solution) between samples without pre- or post- quality control wash run

Run minimum 5 washes: by injecting 20 µL of acetonitrile/isopropanol (1:1, v/v) after batch analysis, followed by control sample of the system.

12. Following analysis remove the LC glass vials containing the samples from the autosampler, replace the open lid caps with closed lid caps and store the remaining solution at -20°C.

13. Quantify the data using the Analyst software or updated versions for quantification released by the provider, e.g. Multiquant (for AB SCIEX instruments) (see Protocol 3 for details).

14. Import the analyst calculated concentrations of eCBs, AA; PEA, OEA and exocannabinoids into an excel sheet. Input the sample number and normalize the values of the Analyst calculated concentrations of the analytes to blood volume, e.g. 20 µL.

15. Perform statistical analysis when sample groups are to be compared.

Note 1: When processing more than 20 samples we use coated (e.g. siliconized) microtiter or deep well plates for LC/MRM analysis to increase the speed of pipetting. Moreover, the storage of the rest samples post analysis is more space effective.

Note 2: The parameters and conditions for LC/MRM are valid for the lab equipment mentioned here. For other MS, LC instruments, or other LC column provider type all these settings have to be optimized accordingly. When polarity switching mode is not available, you can use the rest sample extract to analyze the sample in the additional ionization mode.

### Protocol 3: LC-MRM: Quantification of endocannabinoids, exocannabinoids, arachidonic acid, palmitoylethanolamine and oleoylethanolamine

### 1) Tools

- Analyst software version 1.6.2
- Quantification method 160817_eCBs_THC.qmf

### 2) Quantification procedure

For DBS use following formula to normalize analytes to blood volume:
(C measured analyte (ng/mL) × 50 µLᵥₒₗᵤₘₑ of extracted eCBs)/ 20 µLᵥₒₗᵤₘₑ of blood on the DBS ₛₚₒₜₛ=analyte amount(ng)/ml blood

The following examples and drawings illustrate the present invention without, however, limiting the same thereto.

### BRIEF DESCRIPTION OF THE DRAWINGS

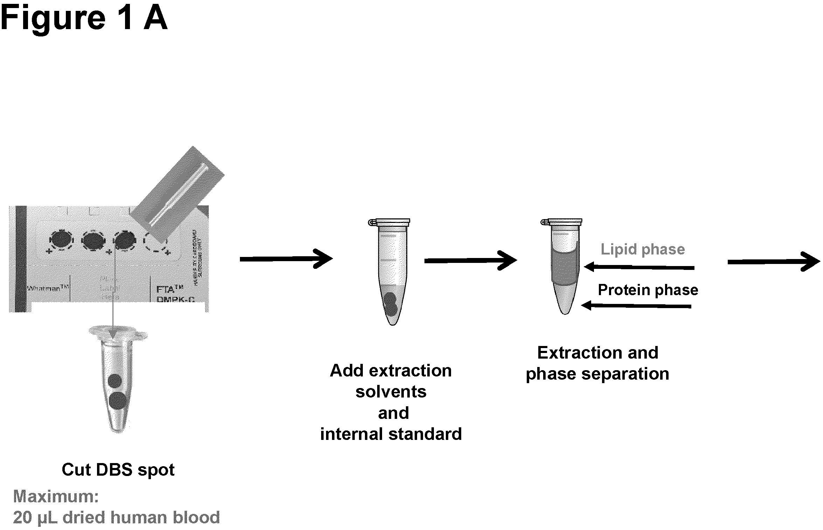

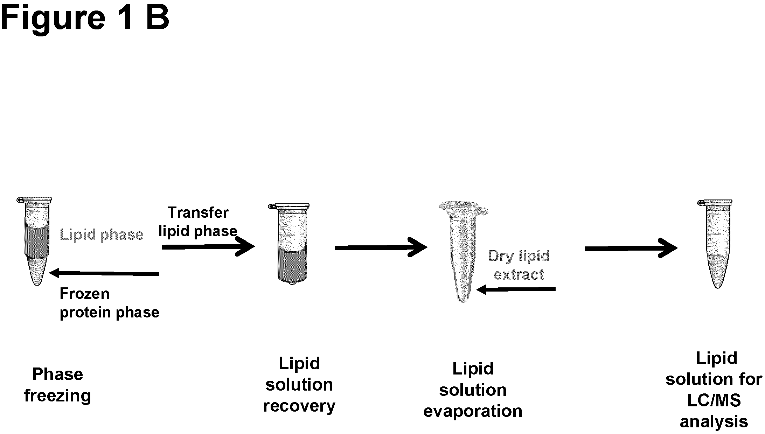

**Figure 1****.** *Scheme of the steps of the detection method of the present invention.*

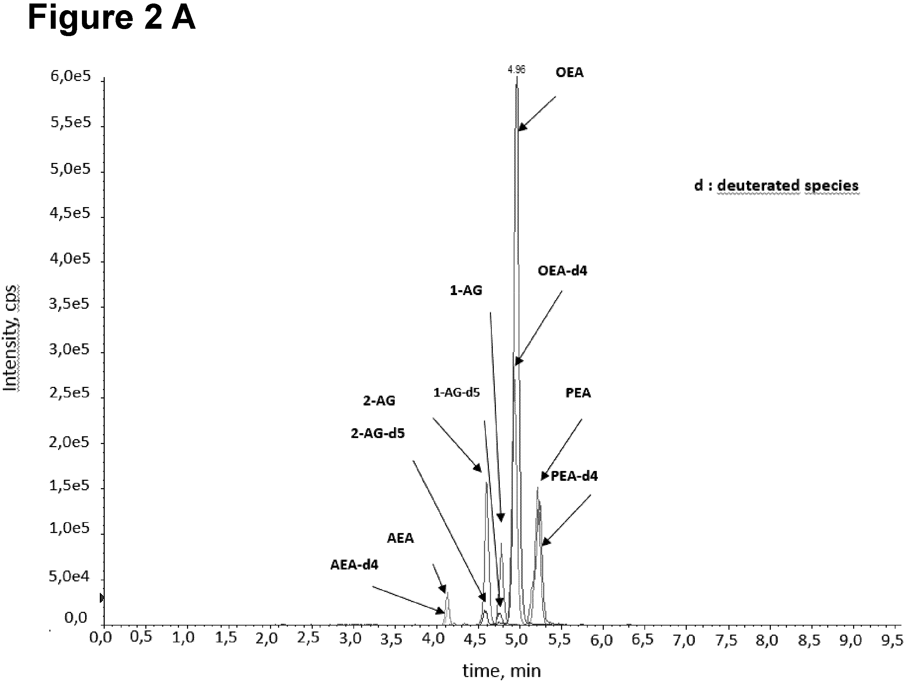

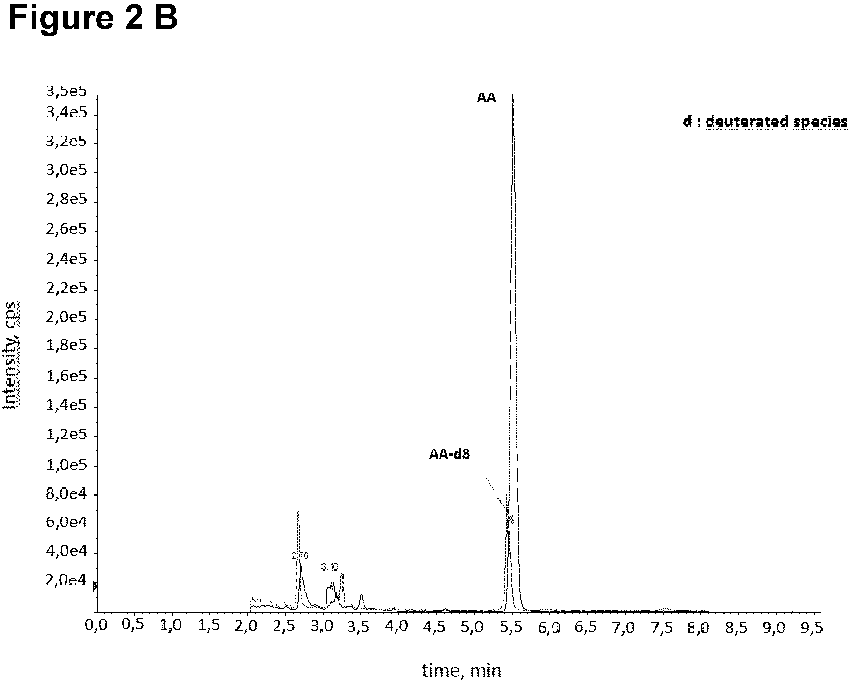

**Figure 2****.** *Example of LC*/*MRM chromatogram obtained from human DBS sample-containing endocannabinoids and endocannabinoid-like lipids, AA, PEA and OEA.*
   **A,** Endocannabinoids AEA and 2-AG, and endocannabinoids-like lipids: OEA and PEA extracted from DBS of human blood, along with corresponding deuterated internal standards.
   **B,** AA (precursor and metabolite of endocannabinodis) extracted from DBS of human blood along with corresponding deuterated internal standard.

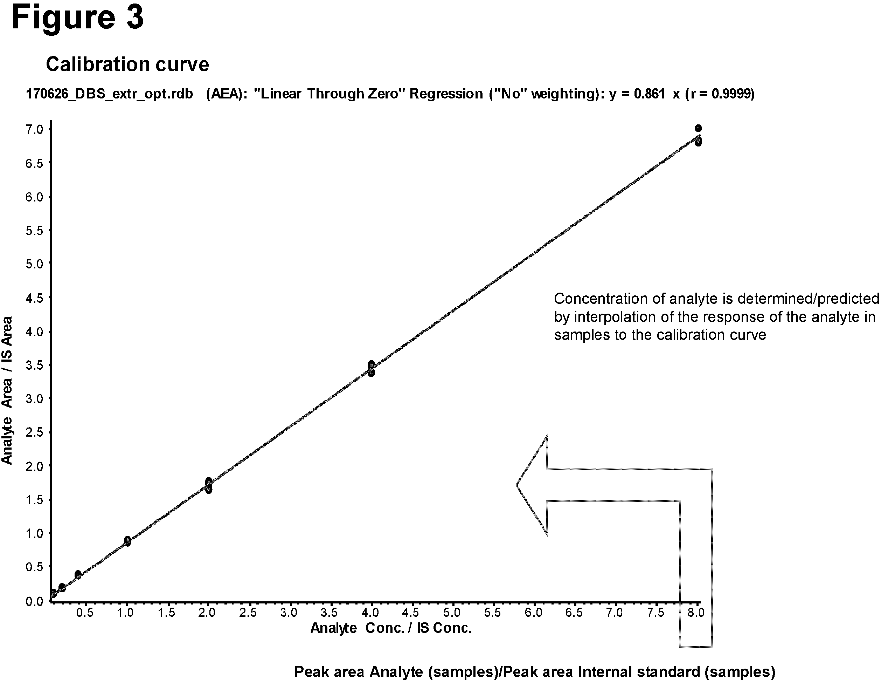

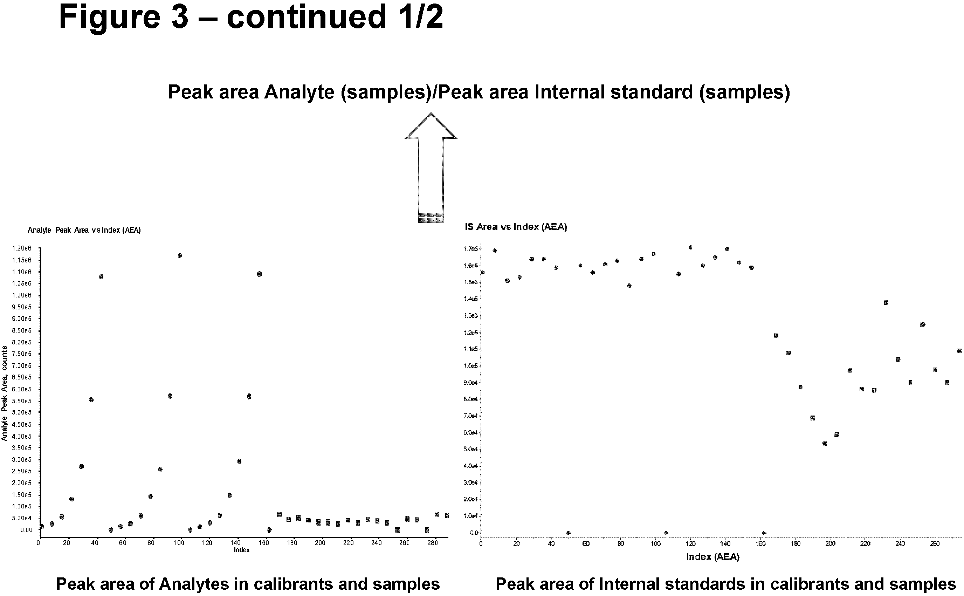

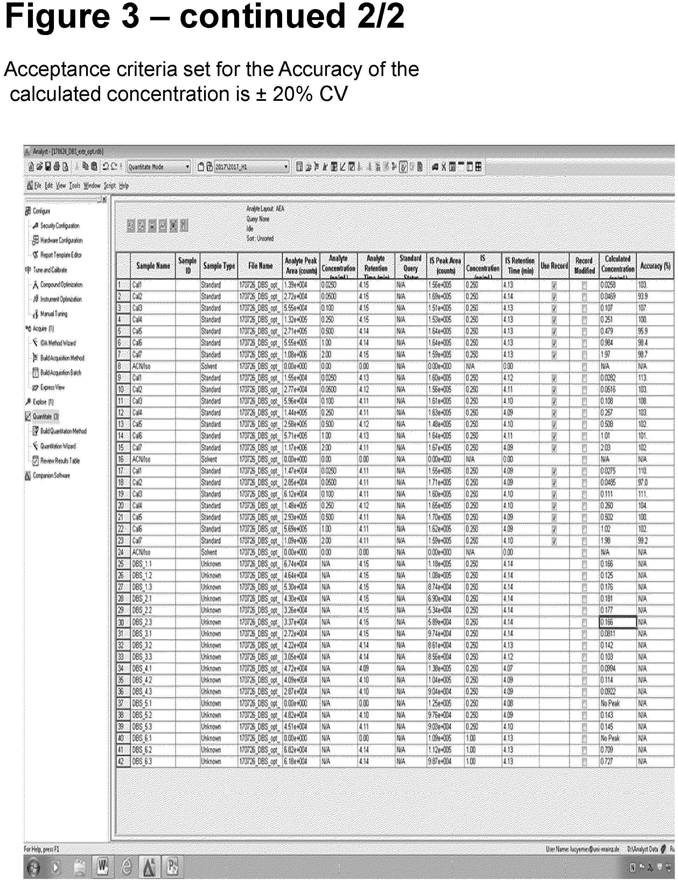

**Figure 3****.** *Quantification.*
   Exerpt of the instrument (QTRAP 5500) quantification sheet, calibration curve and analyte and internal standard responses for AEA in DBS samples and calibrants. Quantification was performed with Analyst software (AB SCIEX),

### EXAMPLES

### Example 1 Material and Methods

All solutions for analyte extraction and LC/MRM analysis have to be of LC/MS grade purity. All solutions for MRM and sample extraction were purchased from Sigma-Aldrich; St. Louis, Missouri, USA).

### a) Internal and calibration standards for all analytes

1. Calibration standards: Anandamide (AEA), 2-arachidonoyl glycerol (2-AG), arachidonic acid (AA), oleoylethanolamine (OEA), palmitoylethanolamine (PEA), 2-arachidonoyl glycerol (1-AG), cannabidiol (CBD), delta-9-tetrahydrocannabinol (THC), 11-hydroxy-delta-9-tetrahydrocannabinol (OH-THC), 11-nor-9-carboxy-delta-9-tetrahydrocannabinol (THCA), were all obtained from BIOMOL Research laboratories Inc. (Plymouth Meeting, PA; USA) or Cayman Chemical (Ann Arbor, USA).

Aliquot the original samples in acetonitrile and store at -20°C.

2. Deuterated standards: AEA-d4, 2-AG-d5, AA-d8, OEA-d4, PEA-d4, 1-AG-d5, CBD-d3, THC-d3, OH-THC-d3, THCA-d3 were all obtained from BIOMOL Research laboratories Inc. (Plymouth Meeting, PA; USA) or Cayman Chemical (Ann Arbor, USA).

The features disclosed in the foregoing description, in the claims and/or in the accompanying drawings may, both separately and in any combination thereof, be material for realizing the invention in diverse forms thereof.

### REFERENCES

Bindila L, Lutz B (2016). Extraction and Simultaneous Quantification of Endocannabinoids and Endocannabinoid-Like Lipids in Biological Tissues. Methods Mol Biol.;1412:9-18. doi: 10.1007/978-1-4939-3539-0_2.
Lerner R, Post J, Loch S, Lutz B, Bindila L Targeting brain and peripheral plasticity of the lipidome in acute kainic acid-induced epileptic seizures in mice via quantitative mass spectrometry. Biochim Biophys Acta. 2017 Feb;1862(2):255-267. doi: 10.1016/j.bbalip.2016.11.008.

## Claims

1. A method of detecting endocannabinoids in blood, comprising the steps of
(a) providing a dried blood spot (DBS) obtained from the blood of a subject; preferably in a DBS collection card,
(b) cutting the dried blood spot or piece(s) thereof and collect it into a sample tube;
(c) adding extraction solvents and an internal standard to the sample tube,
wherein at first a solution of 300 µl ethylacetate/n-hexane in a ratio of 9:1 (v/v) containing further the internal standard are added followed by 300 µl 0.1 M formic acid,
(d) vortexing the mixture in the sample tube overnight while cooling; preferably at about 4°C for about 20 hours,
(e) separating the organic phase from the aqueous phase by centrifuging at 20,000xg for about 10 min while cooling,
preferably at about 4°C,
(f) placing the sample tube on dry ice or in a freezer and letting the aqueous phase freeze;
preferably for about 10 to 20 min, such as 15 min,
(g) transferring the organic phase to a new sample tube while cooling; preferably at about 4°C,
(h) evaporating organic solvents under a N₂ stream and obtaining a dried lipid extract;
(i) reconstituting the dried lipid extract in acetonitrile/water at a ratio of 1:1 (v/v), vortexing the mixture and transferring it to a sample tube suitable for mass spectrometry; and
(j) analyzing the sample mixture obtained in step (i) by performing targeted mass spectrometry using liquid chromatography multiple reaction monitoring (LC/MRM) analysis and simultaneous identifying endocannabinoid(s) and/or metabolite(s) thereof and/or endocannabinoid-related lipid(s).

2. The method of claim 1, wherein in step (b)
- the entire dried blood spot is cut out, preferably when the spot is entirely full (which is equal to 20 µl blood),
- a piece of 2 mm × 3 mm of the dried blood spot is cut out, or
- a piece of 1 mm × 6 mm of the dried blood spot is cut out.

3. The method of claim 1 or 2, wherein in step (b) the cut out dried blood spot or piece(s) thereof are further cut into pieces and then collected in the sample tube.

4. The method of any one of claims 1 to 3, wherein the internal standard used in step (c) comprises a mixture of endoannabinoids and exocannabinoids, which are preferably deuterated, preferably comprising 2-arachidonoyl glycerol (2-AG), anandamide (AEA), 1-arachidonoyl glycerol (1-AG), arachidonic acid (AA), oleoylethanolamine (OEA), palitoylethanolamine (PEA), cannabidiol (CBD), delta-9-tetrahydrocannabinol (THC), 11-hydroxy-delta-9-tetrahydrocannabinol (OH-THC), and 11-nor-carboxy-delta-9-tetrahydro-cannabinol (THCA), more preferably comprising 2-arachidonoyl glycerol (2-AG)-d5, anandamide (AEA)-d4, 1-arachidonoyl glycerol (1-AG)-d5, arachidonic acid (AA)-d8, oleoylethanolamine (OEA)-d4, palitoylethanolamine (PEA)-d4, cannabidiol (CBD)-d3, delta-9-tetrahydrocannabinol (THC)-d3, 11-hydroxy-delta-9-tetrahydrocannabinol (OH-THC)-d3, and 11-nor-carboxy-delta-9-tetrahydro-cannabinol (THCA)-d3.

5. The method of any one of claims 1 to 4, wherein step (d), (e) and/or (g) are carried out in a cold room or at 4°C,
and/or wherein step (f) is carried out at about -20°C,
and/or wherein step (h) is carried out at about 37°C.

6. The method of any one of claims 1 to 5, wherein the dried lipid extract obtained in step (h) is stored at about -20°C before carrying out steps (i) and (j),
and/or wherein in step (i) the sample tube suitable for mass spectrometry is a glass tube or glass vial.

7. The method of any one of claims 1 to 6, wherein the DBS collection card is not pre-treated.

8. The method of any one of claims 1 to 7, wherein the dried blood spot provided in step (a) is obtained at the point-of-care, at home, or in hospitals, clinical research laboratories, blood collection points, medical practices, psychiatry, psychology practices.

9. The method of any one of claims 1 to 8, further comprising
(k) determining the amount of the endocannabinoid(s) and/or metabolite(s) thereof and/or endocannabinoid-related lipid(s),
and/or (1) identifying exocannabinoid(s), and optionally determining their amount.

10. The method of any one of claims 1 to 9, wherein the endocannabinoids are 2-arachidonoyl glycerol (2-AG), anandamide (AEA), 1-arachidonoyl glycerol (1-AG),
and wherein the metabolites of endocannabinoids are arachidonic acid (AA),
and wherein the endocannabinoid-related lipids are oleoylethanolamine (OEA), palitoylethanolamine (PEA),
and wherein the exocannabinoids are cannabidiol (CBD), delta-9-tetrahydrocannabinol (THC), 11-hydroxy-delta-9-tetrahydrocannabinol (OH-THC), 11-nor-carboxy-delta-9-tetrahydro-cannabinol (THCA).

11. The method of any one of claims 1 to 10, wherein the subject is
a patient having a disease or condition which is related to a change of the concentration of endocannabinoid(s) and/or metabolite(s) thereof and/or endocannabinoid-related lipid(s) in the blood or plasma or is a subject suspected of having said disease or condition; or
a subject exhibiting behavioral or psychological disorders and/or behavioral changes which are related to a change of the concentration of endocannabinoid(s) and/or metabolite(s) thereof and/or endocannabinoid-related lipid(s) in the blood or plasma or is a subject suspected of having said disorders.

12. The method of claim 11, wherein said disease or condition or disorder which is related to a change of the concentration of endocannabinoid(s) and/or metabolite(s) thereof and/or endocannabinoid-related lipid(s) in the blood or plasma is a neurological disorder, a psychological or psychiatric disorder, behavioral disorder, eating and/or metabolic disorders,
wherein the neurological disorder is preferably selected from epilepsy, neurodevelopmental diseases, such as Tourette syndrome,
and/or wherein the psychological or psychiatric or behavioral disorder is preferably selected from anxiety, stress-related disorders, post-traumatic stress disorder, addiction, and attention deficit hyperactivity disorder (ADHD).

13. A method for diagnosing and/or monitoring a disease or condition or disorder, which is related to a change of the concentration of endocannabinoid(s) and/or metabolite(s) thereof and/or endocannabinoid-related lipid(s) in the blood or plasma,
comprising the method of any one of claims 1 to 11, wherein the DBS is obtained from the blood of a patient or subject having said disease or condition or disorder or is suspected of having said disease or condition or disorder,
wherein said disease or condition or disorder is preferably as defined in claim 12.

14. The method of claim 13, wherein monitoring the disease or condition or disorder comprises monitoring the treatment of said disease or condition or disorder and/or monitoring the course or progress of said disease or condition or disorder,
wherein preferably the method of any one of claims 1 to 11 is repeated over time.

15. A method for control testing for cannabis use in patients with a disease or condition or disorder related to a change in endocannabinoids and/or related endocannabinoid lipids and treatment thereof,
comprising the method of any one of claims 1 to 11, wherein the DBS is obtained from the blood of a subject which is tested for diagnosis, monitoring and/or therapy response by forensic psychology, medical bodies, clinical researchers, psychologists, or psychiatrists.
